# EUROPÄISCHE PATENTANMELDUNG

(11) **EP 2 479 553 A2**
(43) Veröffentlichungstag der Anmeldung: **25.07.2012**
(21) Anmeldenummer: 12001991.4
(22) Anmeldetag: 02.09.2004
(51) Int. Cl.: G01N 15/14, G01N 15/12, G01N 3/56, G01N 35/10, G01N 33/28

(54) **Vorrichtung zur Partikelmessung mit Einstellen des Eingangsdrucks**

(30) Priorität: 19.09.2003 DE 10343457
(62) Teilanmeldung aus: 04764723.5
(71) Anmelder: Hydac Filtertechnik GmbH, 66280 Sulzbach/Saar (DE)
(72) Erfinder: Busch, Andreas, 66571 Eppelborn (DE); Kleber, Jörg, 66538 Neunkirchen (DE)
(74) Vertreter: Bartels, Martin Erich Arthur

(57) **Zusammenfassung**

Eine Vorrichtung zur Partikelmessung mit einem Partikelzähler (22), bei dem mittels eines Sensors, der auf die Anwesenheit von Partikeln (14) in einer von einem Fluidstrom eines viskosen Mediums (12) durchströmten Meßzone anspricht, ein auswertbares Sensorsignal erzeugt wird, wobei vor Eintritt des Fluidstromes in die Sensor-Meßzone des Partikelzählers (22) mittels einer Einstelleinrichtung (28) der Eingangsdruck für das viskose Medium (12) anpaßbar ist, wobei für die Anpassung des Eingangsdruckes an die temperaturabhängige Viskosität des Mediums (12) die Einstelleinrichtung (28) auf der Abströmseite (24) des Sensors und/oder im Nebenzweig (34) zu diesem angeordnet ist, wobei als Einstelleinrichtung (28) auf der Abströmseite (24) des Sensors ein federbelastetes Rückschlagventil (30) dient und im Nebenzweig (34) eine Drossel (36) vorgesehen ist, ist dadurch gekennzeichnet, dass bei gleichzeitiger Verwendung einer Drossel (36) und eines Rückschlagventils (30) das Rückschlagventil (30) im Fluidkreis in Strömungsrichtung des Mediums (Fluid 12) hinter einer Abzweigstelle (40) angeordnet ist, an der der Nebenzweig (34) mit der Drossel (36) in den Hauptzweig (32) mit dem Sensor des Partikelzählers (22) auf dessen Abströmseite (24) mündet.

## Beschreibung

Die Erfindung betrifft eine Vorrichtung zur Partikelmessung mit einem Partikelzähler, bei dem mittels eines Sensors, der auf die Anwesenheit von Partikeln in einer von einem Fluidstrom eines viskosen Mediums durchströmten Meßzone anspricht, ein auswertbares Sensorsignal erzeugt wird, wobei vor Eintritt des Fluidstromes in die Sensor-Meßzone des Partikelzählers mittels einer Einstelleinrichtung der Eingangsdruck für das viskose Medium anpassbar ist, wobei für die Anpassung des Eingangsdruckes an die temperaturabhängige Viskosität des Mediums die Einstelleinrichtung auf der Abströmseite des Sensors und/oder im Nebenzweig zu diesem angeordnet ist und wobei als Einstelleinrichtung auf der Abströmseite des Sensors ein federbelastetes Rückschlagventil dient und im Nebenzweig eine Drossel vorgesehen ist.

Ein dahingehender Partikelzähler ist beispielhaft in der EP-B-0 427 908 beschrieben. Bei der bekannten Lösung handelt es sich um einen nach dem Abdunklungsverfahren arbeitenden Partikelzähler, insbesondere zum Zählen von opaken Partikeln in einem Fluidstrom eines Hydraulik- oder Schmiersystems, mit einer Lichtschranke, deren Lichtstrahl einen Meßkanal für das Fluid quer zur Kanallängsrichtung durchdringt und einer dem Empfänger der Lichtschranke nachgeschalteten Auswerteelektronik. Die bekannte Lösung ist dadurch charakterisiert, dass die Lichtaustrittsfläche der Lichtschranke durch die Endfläche einer Beleuchtungsphase und die Lichteintrittsfläche durch die Endfläche einer Empfängerphase gebildet ist. Ferner sind die Endfläche der Beleuchtungsphase in einer ersten Begrenzungsfläche des Meßkanals und die konzentrisch zu dieser Endfläche angeordnete Endfläche der Empfängerphase als Meßzone in einer zur ersten Begrenzungsfläche parallelen zweiten Begrenzungsfläche des Meßkanals angeordnet. Die Beleuchtungsphase und die Empfängerphase sind relativ zueinander und zum Meßkanal unbeweglich vor Feuchtigkeit geschützt in einen Sensorkörper eingebettet, der auch den Empfänger der Lichtschranke enthält. Der dahingehende Partikelzähler ist für den rauhen Industrieeinsatz gut geeignet und wenig störanfällig. Ferner ist bei dieser bekannten Lösung vor Eintritt des Fluidstromes in die Sensor-Meßzone des Partikelzählers mittels einer Einstelleinrichtung der Eingangsdruck für das viskose Medium anpassbar. Die bekannte Einstelleinrichtung ist vorzugsweise als Druckabsenkungseinheit ausgebildet und enthält ein Drosselventil sowie ein Druckventil. Ferner ist der Sensor eingangsseitig mit einer fluidführenden Leitung mit der Druckabsenkungseinheit als Einstelleinrichtung verbunden.

Durch die DE-C-197 35 066 ist darüber hinaus ein Auswerteverfahren für einen dahingehenden Partikelzähler und eine Vorrichtung zum Durchführen des Verfahrens bekannt, wobei ein Sensor des Partikelzählers auf die Anwesenheit von Partikeln in einer von einem Fluidstrom durchströmten Meßzone anspricht, wodurch ein Sensorsignal erzeugt wird, das mittels einer Signalverarbeitungseinrichtung aufbereitet und unter Berücksichtigung zumindest eines Kalibrierfaktors in einen Anzeigewert überführt wird, wobei die Aufbereitung des Sensorsignals so durchgeführt wird, dass innerhalb eines vorgegebenen Zeitraumes die individuellen Verweilzeiten der Partikel in der Meßzone ermittelt werden und wobei durch Summation der Verweilzeiten ein Summensignal gebildet und dieses unter Berücksichtigung des zumindest einen Kalibrierfaktors zur Darstellung des Anzeigewertes benutzt wird. Die Vorrichtung zum Durchführen des Meßverfahrens weist dabei eine Signalverarbeitungseinrichtung mit einer Komparatorschaltung, einem Taktgeber und mit einer Summiereinrichtung auf, beispielsweise in Form eines Impulszählers zur Bildung des Summensignals.

Durch die DE-A-41 10 231 ist eine weitere gattungsgemäße Meßeinrichtung zum Bestimmen des Schmutzpartikelanteils von Flüssigkeiten bekannt, insbesondere Hydraulikölen, bei der kontinuierlich Flüssigkeit in eine Strömungsleitung der Meßeinrichtung (Sensor) abziehbar und über diese einem Schmutzpartikel-Zählgerät zum Erzeugen von Partikelmeßwerten zuführbar ist. Die bekannte Lösung zeichnet sich durch ein in der Strömungsleitung angeordnetes, von der abgezogenen Flüssigkeit durchströmtes Durchflußmeßgerät (Sensor) zum Erzeugen von Durchflußmeßwerten und durch einen die Partikel- und Durchflußmeßwerte kombinierend verarbeitenden Rechner zum Berechnen eines auf eine vorbestimmbare Volumeneinheit bezogenen Verschmutzungsgrades aus. Hierdurch lassen sich auf einfache Weise kleinere Durchflußschwankungen berücksichtigen, die sofort korrigierend in das Meßergebnis eingehen. Vergleichbar der erstgenannten gattungsgemäßen Lösung nach der europäischen Druckschrift sind ferner auf der Eingangs- oder Eintrittsseite des Sensors ein Rückschlagventil angeordnet sowie ein zusätzlich stromab hiervon angeschlossener Druckspeicher und ein Durchflußregler. Mit der dahingehenden eingangsseitigen Druck-Einstelleinrichtung für das viskose Medium werden eine Betriebsunterbrechung bei vorübergehender Unterschreitung eines bestimmten Systemmindestdruckes vermieden und eine Anpassung des Durchflusses an den Nennwert des Schmutzpartikel-Zählgerätes ermöglicht.

Bei den bekannten Lösungen hat es sich gezeigt, dass der Volumenstrom des zu analysierenden Fluidmediums mittels eines Partikelzählers als Sensoreinrichtung innerhalb eines definierten Bereiches liegen muß, z.B. zwischen 20 bis 200 ml/min. Dabei ist der untere Grenzwert durch die Tatsache vorgegeben, dass bei Unterschreiten desselben die Partikel im Fluidstrom sedimentieren, und zwar auf der Einströmseite (Zuleitung) zum Sensor, was zum einen mit wachsender Sedimentierung zu Hemmnissen bei der Messung führt, und zum anderen, da die sedimentierten Partikel als Verschmutzung von dem Sensor nicht mehr detektierbar sind, kommt es zu Falschaussagen und mithin zu einer fälschlichen Bewertung der Qualität des Fluids. Die genannte obere Volumenstromgrenze von bis zu 200 ml/min. ergibt sich daraus, dass die von den Schmutzpartikeln erzeugten Signale in der Meßzone des Sensors mit zunehmender Strömungsgeschwindigkeit immer kürzer werden und durch die beschränkte Bandbreite der nachgeschalteten Meßschaltungen werden die Signale entsprechend gedämpft und somit die Partikelgröße falsch bestimmt.

Ein weiterer Störfaktor für eine optimale Messung ergibt sich aufgrund der temperaturabhängigen Viskosität des Fluids (Mediums), die bei vielen, insbesondere industriellen Anwendungen stark schwanken kann. Insbesondere bei Installationen im Freien kann temperaturbedingt die Viskosität des Fluids derart stark schwanken, dass bei einem fest vorgegebenem Eingangsdruck am Partikelzähler der resultierende Volumenstrom zum Teil außerhalb des noch zulässigen Bereiches liegt, was gleichfalls zu unbrauchbaren Meßergebnissen führt. Im Stand der Technik sind zwar bereits konventionelle Mittel zur Regelung des Volumenstromes vorgeschlagen worden, um dem dahingehenden Problem zu begegnen. Die hierzu häufig eingesetzten Schieberventile sind aber zum einen sehr verschmutzungsempfindlich, generieren hohe Kosten und benötigen relativ hohe Eingangsdrücke, um überhaupt betriebstauglich zu sein.

Durch die JP 63-144235 A ist eine gattungsgemäße Vorrichtung zur Partikelmessung bekannt, bei der mittels einer umschaltbaren Drosseleinrichtung mit mehreren Drosseln, die auf der Abströmseite des Sensors angeordnet sind, eine Umschaltung erfolgt, bei der für eine Grobmessung zu Beginn eine hohe Fließgeschwindigkeit erwünscht ist und später für eine Feinmessung eine niedrige Fließgeschwindigkeit.

Ausgehend von diesem Stand der Technik liegt der Erfindung die Aufgabe zugrunde, die bekannten Vorrichtungen zur Partikelmessung dahingehend weiter zu verbessern, dass die beschriebenen Nachteile nicht mehr auftreten, insbesondere dass bei geringen Herstellkosten eine funktionssichere Vorrichtung erhalten ist, die verbesserte Messungen für einen mit Partikeln (Verschmutzungen) beladenen Fluidstrom liefert. Eine dahingehende Aufgabe löst eine Vorrichtung mit den Merkmalen des Patentanspruches 1 in seiner Gesamtheit.

Dadurch, dass für die Anpassung des Eingangsdruckes an die temperaturabhängige Viskosität des Mediums die Einstelleinrichtung auf der Abströmseite des Sensors und/oder im Nebenzweig zu diesem angeordnet ist, besteht die Möglichkeit, in Abhängigkeit von der Viskosität des Fluids (Medium) einen für die Partikelmessung geeigneten Eingangsdruck am Partikelzähler mit einem Sensor zu erzeugen. Auf diese Art und Weise läßt sich in kostengünstiger und funktionssicherer Form die temperaturabhängige Viskosität des Fluids in weiten Bereichen derart kompensieren, dass der Volumenstrom in weiten Bereichen konstant bleibt mit der Folge, dass dieser definierte Bereiche einhält, für die der jeweilige Partikelzähler mit seinem Sensor meßtechnisch ausgelegt ist. Sofern innerhalb des zu analysierenden Fluids sich als Verschmutzungspartikel Gase, wie Luft oder Stickstoff, aufhalten sollten, die gleichfalls die Viskosität des Fluids für eine exakte Partikelmessung nachteilig beeinflussen können, ist über die Einstelleinrichtung jedenfalls der Eingangsdruck für das Fluidmedium derart vorgebbar, dass dahingehende gasförmige Partikel im Fluid vor der Messung gelöst werden, so dass diese vom Partikelzähler auch nicht mehr als schädliche Verschmutzungsanteile erkannt werden, die ansonsten das Meßergebnis ebenfalls verfälschen würden.

Dabei dient als Einstelleinrichtung auf der Abströmseite des Sensors ein federbelastetes Rückschlagventil und im Nebenzweig eine Drossel. Die dahingehenden Einstelleinrichtungen lassen sich funktionssicher betreiben und sind kostengünstig in einer Vielzahl von Ausführungsformen auf dem Markt frei erhältlich.

Bei gleichzeitiger Verwendung einer Drossel und eines Rückschlagventils ist das Rückschlagventil im Fluidkreis in Strömungsrichtung des Mediums hinter einer Abzweigstelle angeordnet, an der der Nebenzweig mit der Drossel in den Hauptzweig mit dem Sensor des Partikelzählers auf dessen Abströmseite mündet. Hierdurch läßt sich über das genannte federbelastete Rückschlagventil der Eingangsdruck für den Partikelzähler an die temperaturabhängige Viskosität anpassen, mit der Folge, dass der Druck in der Meßzelle angehoben ist, um die im System befindlichen, die Messung störenden Luftblasen besser im Fluid lösen zu können.

Bei einer bevorzugten Ausführungsform der erfindungsgemäßen Vorrichtung ist in Fluidrichtung vor der Drossel im Nebenzweig zu dem Sensor eine Filtereinheit angeordnet. Mit der dahingehenden Filtereinheit im Nebenstrom lassen sich Verschmutzungen aus dem Fluidkreis herausfiltrieren, so dass insoweit der Partikelzähler entlastet ist. Aufgrund des Wegfalls einer Vielzahl zum Sedimentieren neigender Partikel durch Filtration, läßt sich dann die dahingehende erfindungsgemäße Vorrichtung länger einsetzen.

Bei einer weiteren besonders bevorzugten Ausführungsform der erfindungsgemäßen Vorrichtung weist die Einstelleinrichtung auf der Einströmseite des Sensors ein Leitungsstück vorgebbarer Länge auf. Vorzugsweise kann dabei das Leiterstück zwischen einer weiteren Abzweigstelle des Nebenzweiges mit der Drossel und dem Sensor auf seiner Einströmseite angeordnet sein. Auf diese Art und Weise lassen sich im Fluid mitgeführte Gase im Fluid wieder lösen und da das Gas (Luft) unter Druck nicht schlagartig im Fluid gelöst wird, dient die vorgebbare Länge des Leitungsstückes als Beruhigungsstrecke, in der sich die Luft wieder im Fluid lösen kann. Würde man die Gas- oder Luftblasen durch den Sensor schicken, würde der optische Partikelzähler aufgrund der verschiedenen Brechungsindizies zwischen Luft und Medium (Öl) den Gas- oder Lufteintrag als schädliche Verschmutzung für das Fluid detektieren, was zu einer Fehleinschätzung über die Qualität des Fluidmediums führen würde.

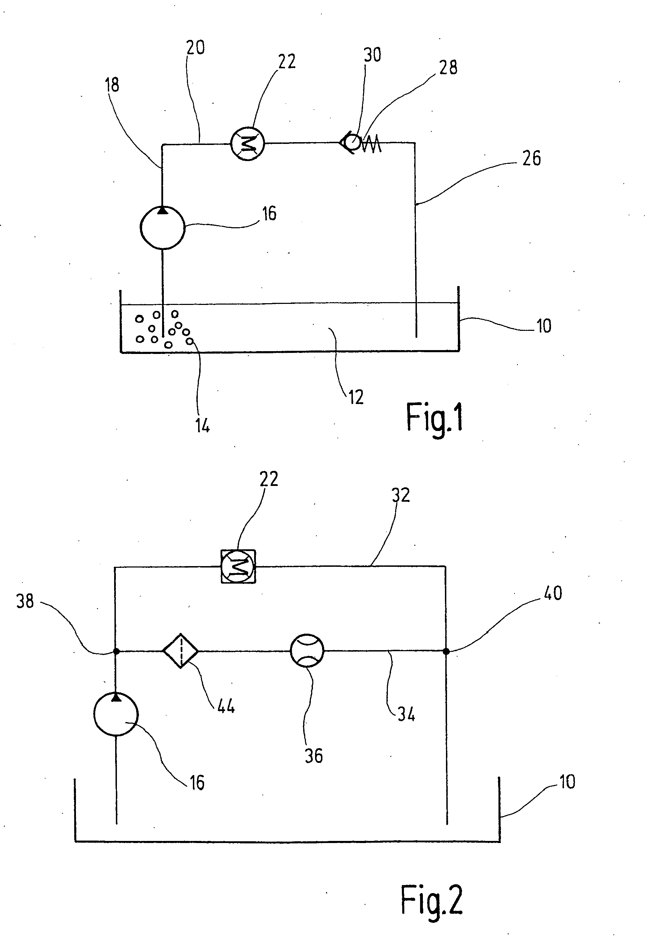

Sofern vorzugsweise der Sensor des Partikelzählers mit einer Temperier-, insbesondere Heizeinrichtung versehen ist, besteht die Möglichkeit, auch bei tiefen Außentemperaturen das Medium derart zu erwärmen, dass ein nennenswerter und gewünschter Volumenstrom zustande kommt. Vorzugsweise ist dabei die Temperier- oder Heizvorrichtung außerhalb des eigentlichen Sensors des Partikelzählers angeordnet, um dergestalt eine negative Beeinflussung des Sensors mit seiner Meßzone vermeiden zu helfen. Im folgenden wird die erfindungsgemäße Vorrichtung anhand verschiedener Ausführungsformen nach der Zeichnung näher erläutert. Dabei zeigen in prinzipieller und nicht maßstäblicher Darstellung die
- Fig.1 bis 4: vier verschiedene Ausführungsformen der erfindungsgemäßen Sensorvorrichtung anhand von entsprechenden Schaltplänen.

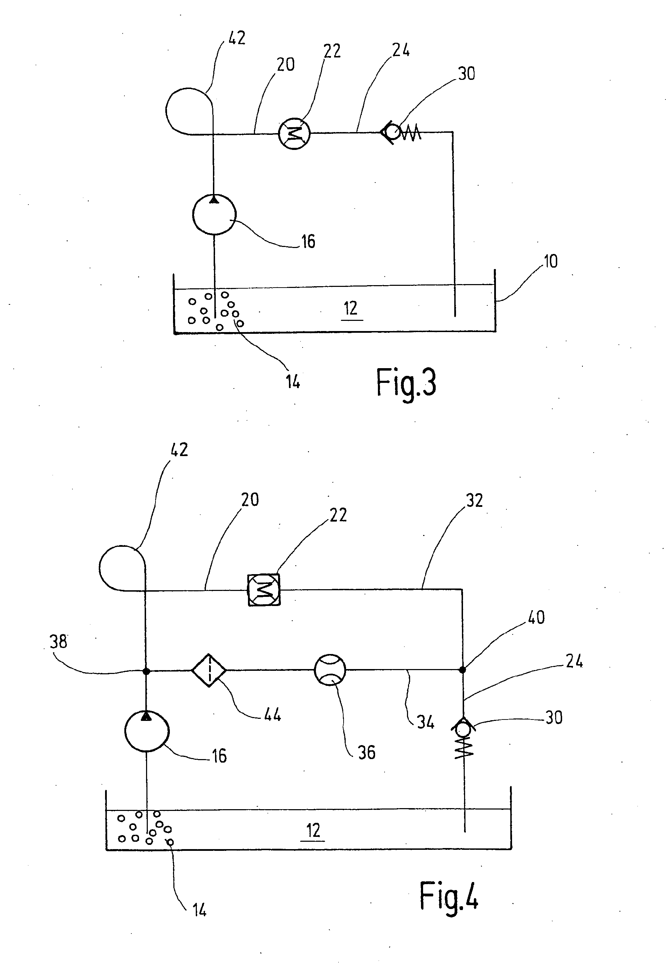

Die erfindungsgemäße Sensorvorrichtung ist nur in Fig. 4 gezeigt. Die übrigen Figuren dienen lediglich der Erläuterung des Hintergrundes der Erfindung.

Beginnend mit der Fig.1 weist die dort gezeigte Vorrichtung einen Fluidtank 10 auf, dessen Tankinhalt in Form eines Fluids 12, beispielsweise in Form eines Hydraulikmediums, dem Umgebungsdruck ausgesetzt ist. Innerhalb des Fluids 12 können beispielsweise durch Aufschäumvorgänge beim Betrieb einer Hydraulikanlage od. dgl. Gasblasen, insbesondere in Form von Luftblasen 14, eingelagert sein. Über eine als Konstantpumpe ausgebildete Hydraulikpumpe 16 wird Fluid 12 aus dem Tank 10 über eine Fluidleitung 18 entnommen und auf die Einströmseite 20 des Partikelzählers 22 gebracht. Auf der Abströmseite 24 des Partikelzählers 22 ist in eine zugehörige Fluidleitung 26, die zurück zum Tank führt, eine Einstelleinrichtung 28 geschaltet.

Als Partikelzähler kann beispielsweise ein solcher verwendet werden, wie er in der EP-B-0 427 908 beschrieben ist, und als Auswerteverfahren für die erhaltenen Sensorsignale kann beispielsweise ein solches dienen, wie es in der DE-C-197 35 066 beschrieben ist. Der dahingehende Offenbarungsgehalt der beiden genannten Dokumente wird insoweit vollumfänglich auch für die vorliegende Patentanmeldung in Anspruch genommen, wobei für die genannte Vorrichtung auch andere, nicht näher beschriebene Partikelzählereinrichtungen und Auswerteverfahren Anwendung finden können.

Mittels der genannten Einstelleinrichtung 28 ist vor Eintritt des Fluidstromes in die Sensor-Meßzone des Partikelzählers 22 der Eingangsdruck für das viskose Medium 12 (Fluid) an die temperaturabhängige Viskosität anpaßbar. Demgemäß ist es möglich, über die Einstelleinrichtung 28 den Volumenstrom des zu analysierenden Fluids für den Partikelzähler 22 in dessen vorgebbaren Bereichsgrenzen, beispielsweise zwischen 20 bis 200 ml/min., zu halten. Durch den vorgebbaren Volumenstrom läßt sich somit die Gefahr ausschließen, dass aufgrund eines zu geringen Volumenstromes die Verschmutzungspartikel sedimentieren können und dass bei zu hohen Volumenströmen aufgrund von Signaldämpfung die Partikelgröße falsch bestimmt wird. Ferner erlaubt die Einstelleinrichtung 28, dass die in der Fig.1 dargestellten Luftblasen 14 früher in Lösung gehen, und zwar im Bereich des zu führenden Fluids innerhalb der Fluidleitung 18, so dass insoweit der Partikelzähler 22 nicht im Sinne einer Fehlmessung dahingehende unschädliche Luftblasen 14 als Verschmutzungspartikel erkennt, was ansonsten zu einer schlechten Bewertung des eingesetzten Hydraulikmediums führen würde. Als Einstelleinrichtung 28 für die Lösung nach der Fig.1 dient dabei ein federbelastetes Rückschlagventil 30, das funktionssicher und kostengünstig zu betreiben bzw. zu erhalten ist. Je nachdem, welchen Volumenstrom man hier dem Partikelzähler 22 zuführen will, sind dann die entsprechenden Nennweiten des Rückschlagventils 30 sowie dessen Federstärken auszuwählen und vorzugeben.

Bei den nachfolgenden Ausführungsformen ist der Grundaufbau nach der Fig.1 im wesentlichen erhalten, so dass insoweit für die nachfolgenden Ausführungsbeispiele dieselben Bezugszeichen verwendet werden wie in der Fig.1. Ferner werden die nachfolgenden Ausführungsformen nur noch insofern erläutert, als sie sich im wesentlichen von der ersten Ausführungsform nach der Fig.1 unterscheiden.

Bei der Ausführungsform nach der Fig.2 ist in einem Hauptzweig 32 wiederum der Partikelzähler 22 geschaltet und im Nebenschluß zu dem Partikelzähler 22 und zum Hauptzweig 32 ist in diesen ein Nebenzweig 34 mit einer Drossel 36 geschaltet. Dabei mündet die fluidführende Leitung des Nebenzweiges 34 in Form zweier Abzweigstellen 38,40 in den Hauptzweig 32 ein. Die eingesetzte Drossel 36 ist dadurch charakterisiert, dass sie eine Querschnittsverengung aufweist, bei der die Länge ihrer Öffnung wesentlich größer ist als der Durchmesser. Aufgrund der beim Durchströmen entstehenden Reibung ist der Durchfluß durch die Drossel 36 viskositätsabhängig. Durch die dahingehende Anordnung ist eine Art Stromteiler realisiert und aufgrund der gleichen Kennliniencharakteristik der viskositätsabhängigen Drossel 36 läßt sich ein Eingangsdruck für den Partikelzähler 22 dergestalt erzeugen, dass über den Stromteiler beispielsweise neun Teile des Fluidstromes die Drossel 36 durchqueren und ein Teil den Partikelzähler (Verschmutzungssensor) 22.

Aufgrund des gleichbleibenden Aufteilungsverhältnisses, hervorgerufen durch die Stromteilercharakteristik an der ersten Abzweigstelle 38, läßt sich der Partikelzähler 22 für geringe Volumenströme konstruktiv klein aufbauen, was hilft, Einbauraum zu sparen sowie Kosten für das Zurverfügungstellen des Partikelzählers 22. Auch mit der dahingehenden Lösung lassen sich wiederum meßtechnisch schädliche Gase im Fluidstrom lösen, insbesondere wenn gemäß der Darstellung nach der Fig.4 in Strömungsrichtung hinter der weiteren zweiten Abzweigstelle 40 das federbelastete Rückschlagventil 30 gemäß der Darstellung nach der Fig.1 setzt. Ferner läßt sich der Vorgang des Gaslösens im Fluid noch weiter verbessern, wenn man gemäß den Darstellungen nach den Fig.3 und 4 auf der Einströmseite 20 zwischen Hydropumpe 16 und Partikelzähler 22 ein Leitungsstück 42 in der Art einer Beruhigungsstrecke einsetzt. Durch die dahingehende Anordnung lassen sich die im Stand der Technik bekannten Sammelbehälter vermeiden, die zum einen dazu dienen, die Gasblasen zu sammeln und an die Umgebung abzugeben, und zum anderen im Sinne eines hydraulischen Umlaufs das Ein-und Ausströmen des zu transportierenden Fluids zu ermöglichen. Sieht man bei den bekannten Lösungen vor, dass die Strömungsgeschwindigkeit des Fluids 12 im hydraulischen Kreis kleiner ist als die Geschwindigkeit, mit der die gesammelten Gasblasen im Behälter aufsteigen, ist dergestalt eine Entgasung erreicht und der in den hydraulischen Kreis nachgeschaltete Partikelzähler kommt mit den meßtechnisch schädlichen Gas- oder Luftblasen erst gar nicht in Berührung. Die dahingehend bekannte Lösung ist aber apparatetechnisch aufwendig in der Realisierung und im Hinblick auf die genannten kleinen Strömungsgeschwindigkeiten für das einzusetzende Fluid ist die Effizienz der bekannten Anlagen merklich reduziert.

Besonders vorteilhaft ist es, den im Nebenzweig 34 geführten Hauptstrom an Fluid über eine Filtereinheit 44 (vgl. Fig.2 und 4) abzureinigen, um dergestalt den Partikelschmutz im hydraulischen Kreis ohnehin niedrig zu halten. Mit den Ausgestaltungen der Vorrichtung in den Fig.1 bis 4 ist es möglich, zeitnah, also On-Line, Verschmutzungen beispielsweise innerhalb von Getriebeölen bei Windkraftanlagen od. dgl., zu bestimmen. Mit der Vorrichtung ist es mithin möglich, On-Line den Verschleißzustand eines Getriebes anhand des Verlaufs der Feststoffverschmutzung im Getriebeöl zu überwachen und zu bewerten, was es erlaubt, die Standzeit von Windkraftanlagen und deren Komponenten deutlich zu erhöhen. Die vorstehend verschiedentlich beschriebene Volumenstromteilung führt insbesondere dann zum gewünschten Effekt, wenn ein konstanter Volumenstrom, beispielsweise mittels einer entsprechenden Konstant-Pumpe, zur Verfügung gestellt wird.

## Patentansprüche

1. Vorrichtung zur Partikelmessung mit einem Partikelzähler (22), bei dem mittels eines Sensors, der auf die Anwesenheit von Partikeln (14) in einer von einem Fluidstrom eines viskosen Mediums (12) durchströmten Meßzone anspricht, ein auswertbares Sensorsignal erzeugt wird, wobei vor Eintritt des Fluidstromes in die Sensor-Meßzone des Partikelzählers (22) mittels einer Einstelleinrichtung (28) der Eingangsdruck für das viskose Medium (12) anpaßbar ist, wobei für die Anpassung des Eingangsdruckes an die temperaturabhängige Viskosität des Mediums (12) die Einstelleinrichtung (28) auf der Abströmseite (24) des Sensors und/oder im Nebenzweig (34) zu diesem angeordnet ist und wobei als Einstelleinrichtung (28) auf der Abströmseite (24) des Sensors ein Ventil, vorzugsweise ein federbelastetes Rückschlagventil (30), dient und im Nebenzweig (34) eine Drossel (36) vorgesehen ist, **dadurch gekennzeichnet, dass** bei gleichzeitiger Verwendung einer Drossel (36) und eines Ventils (30) das Ventil (30) im Fluidkreis in Strömungsrichtung des Mediums (Fluid 12) hinter einer Abzweigstelle (40) angeordnet ist, an der der Nebenzweig (34) mit der Drossel (36) in den Hauptzweig (32) mit dem Sensor des Partikelzählers (22) auf dessen Abströmseite (24) mündet.

2. Vorrichtung nach Anspruch 1, **dadurch gekennzeichnet, dass** in Fluidrichtung vor der Drossel (36) im Nebenzweig (34) zu dem Sensor des Partikelzählers (22) eine Filtereinheit (44) angeordnet ist.

3. Vorrichtung nach Anspruch 1 oder 2, **dadurch gekennzeichnet, dass** die Einstelleinrichtung (28) auf der Einströmseite (20) des Sensors ein Leitungsstück (42) vorgebbarer Länge aufweist.

4. Vorrichtung nach Anspruch 3, **dadurch gekennzeichnet, dass** das Leitungsstück (42) zwischen einer weiteren Abzweigstelle (38) des Nebenzweiges (34) mit der Drossel (36) und dem Sensor auf seiner Einströmseite (20) angeordnet ist.

5. Vorrichtung nach einem der Ansprüche 1 bis 4, **dadurch gekennzeichnet, dass** der Sensor des Partikelzählers (22) mit einer Temperier-, insbesondere Heizeinrichtung versehen ist.
